# EUROPEAN PATENT SPECIFICATION

(11) **EP 4 262 572 B1**
(45) Date of publication and mention of the grant of the patent: **02.07.2025**
(21) Application number: 21732576.0
(22) Date of filing: 09.06.2021
(51) Int. Cl.: A61B 17/00, A61B 17/32

(54) **IRRIGATION SLEEVE FOR ULTRASONIC SURGICAL ASSEMBLY**
BEWÄSSERUNGSHÜLSE FÜR CHIRURGISCHE ULTRASCHALLANORDNUNG
MANCHON D'IRRIGATION POUR L'ENSEMBLE CHIRURGICAL À ULTRASONS

(30) Priority: 18.12.2020 US 202063127802 P; 07.06.2021 US 202163197773 P
(43) Date of publication of application: 25.10.2023
(73) Proprietor: Stryker European Operations Limited, Carrigtwohill, Co. Cork T45 HX08 (IE)
(72) Inventor: GRAS, Guillaume, 3053 Muenchenbuchsee (CH); HENDERSON, Daniel, Cork (IE); MCCARTHY, Conor, Cork (IE)
(74) Representative: Röthinger, Rainer
(86) International application number: PCT/IB2021/055077
(87) International publication number: WO 2022/130035

(56) References cited:
- WO-A1-2020/068756
- US-A- 5 695 510
- US-A1- 2013 023 866
- US-A1- 2015 073 457
- US-A1- 2017 340 339
- US-A1- 2017 348 051
- US-A1- 2020 205 850
- US-B1- 6 177 755

## Description

### BACKGROUND

A cooling system for a surgical handpiece to provide fluid flow to the surgical tip and to the internal electronic components to prevent temperatures that may damage or make the patient or surgeon uncomfortable is known from US 2013/0023866 A1. Tip directed gas further controls the tip-tissue interface by displacing blood or serum from the incision point, increasing precision and diminishing coagulum build-up. In the alternative, cooling fluid may be used to quench tissue being treated.

A system for use with a microwave antenna as disclosed in US 2017/0348051 A1 includes a microwave antenna configured to deliver microwave energy from a power source to tissue and a sensor module in operative communication with the power source and configured to detect a reflectance parameter. The system further includes a jacket adapted to at least partially surround the microwave antenna to define a fluid channel between the jacket and the microwave antenna. A plurality of fluid distribution ports are defined through the jacket and are in fluid communication with the fluid channel to permit the flow of fluid through the jacket. The system further includes a fluid pumping system operably coupled to the power source and configured to selectively provide cooling fluid to the fluid channel for distribution through the fluid distribution ports based on the reflectance parameter.

WO 2020/068756 A1 discloses an ultrasonic surgical handpiece assembly comprising a surgical handpiece for use with an irrigation sleeve and ultrasonic tip. The surgical handpiece may comprise a piezoelectric transducer disposed within a housing and configured to manipulate the ultrasonic tip. One or more lumens and/or a flex circuit including an antenna may be disposed within the surgical handpiece housing. The lumen(s) may be configured to provide irrigation and/or aspiration to the irrigation sleeve and/or ultrasonic tip. The irrigation sleeve may comprise a second antenna configured to communicate with the ultrasonic handpiece antenna. The irrigation sleeve may further comprise an alignment and/or coupling feature configured to removably secure the irrigation sleeve to the housing and orient the second antenna relative to the ultrasonic handpiece antenna. The irrigation sleeve may further comprise a lumen for supplying irrigation and/or aspiration to the ultrasonic tip.

### SUMMARY

An irrigation sleeve for an ultrasonic surgical assembly is defined in claim 1 and an ultrasonic surgical assembly comprising the irrigation sleeve is defined in claim 4.

An exemplary ultrasonic surgical assembly to provide air cooling of an ultrasonic tip is disclosed. The ultrasonic surgical assembly includes an ultrasonic tip and an irrigation sleeve. The ultrasonic tip comprises a shaft and a cutting portion and has a first side and a second side. The first side is substantially planar and extends from a proximal end to a distal end. The second side is substantially planar, is disposed opposite the first side, and extends from the proximal end to the distal end. The cutting portion includes a cutting head, which is disposed at the distal end. The ultrasonic tip is removably coupled to a horn. The shaft further comprises a longitudinal axis. The irrigation sleeve has a distal region and a proximal region and defines a lumen that extends along the longitudinal axis. The irrigation sleeve at least partially surrounds the shaft and defines an inlet aperture. The irrigation sleeve further comprises a first conduit in fluid communication with the lumen. The first conduit has an outlet aperture and is configured for being connected to a liquid source. The ultrasonic tip further comprises a sealing member coupled to its outer surface and further defines a first bore and a second bore. The first bore defines an air inlet disposed proximal to the sealing member, and the second bore extends from the proximal end of the shaft to the first bore to form a fluid path between the first bore and the second bore.

A second ultrasonic surgical assembly corresponding to the ultrasonic surgical assembly of claim 4 is disclosed. The assembly includes an ultrasonic instrument, an ultrasonic tip, and an irrigation sleeve. The ultrasonic instrument comprises a housing, a transducer, and a horn. The housing comprises a proximal portion and a distal portion. The transducer is at least partially disposed within the housing. The horn is coupled to the transducer. The ultrasonic tip comprises a shaft and is removably coupled to the horn. The irrigation sleeve defines a lumen and comprises a body, a sheath, and an irrigation conduit. The body is releasably coupled to the distal portion of the housing and has a distal region and a proximal region. The body defines a helical groove which at least partially surrounds the shaft when the ultrasonic tip is in the lumen of the irrigation sleeve. The sheath is coupled to and disposed over a portion of the body to surround at least a full revolution of the helical groove. The sheath has a proximal end and an opposing distal end. The irrigation conduit is disposed within the helical groove for conveying irrigation fluid and defines an inlet aperture and an outlet aperture. The inlet aperture is disposed at the proximal region of the body, and an outlet aperture is disposed at a distal region of the body. The irrigation fluid enters the irrigation conduit at the inlet aperture and exits the irrigation conduit at the outlet aperture.

An exemplary ultrasonic tip is disclosed that comprises a shaft and a cutting portion. The shaft defines a longitudinal axis. The cutting portion defines a first side that is substantially planar and a second side that is substantially planar and includes a cutting head. The cutting portion includes a base portion having a transverse dimension between the first side and the second side that extends perpendicular to a longitudinal axis of the cutting head. The cutting portion further includes a tapered portion that comprises a bevel and that extends from the base portion to a cutting edge. The cutting edge comprises a length and has a U-shaped profile having a first leg portion, a second leg portion, and an arcuate shaped distal portion. The first leg portion and the second leg portion are parallel to one another. Moreover, the largest cross-sectional area of the ultrasonic tip defines a cross-sectional area of a first slice. A cross-sectional area of a second slice is defined at a location 20 mm proximal to a distal end of the ultrasonic tip. The second slice and the first slice are each perpendicular to the longitudinal axis of the shaft. The cross-sectional area of the second slice is 16.7 - 20 % of the cross-sectional area of the first slice.

An exemplary second ultrasonic surgical assembly to provide air cooling of an ultrasonic tip is disclosed. The ultrasonic surgical assembly comprises an ultrasonic instrument, an ultrasonic tip, an irrigation sleeve, and a sealing member. The ultrasonic instrument has a proximal region and a distal region and comprises a housing, a transducer, and a horn. The housing comprises a proximal portion and a distal portion. The transducer is at least partially disposed within the housing. The horn is coupled to the transducer, which is configured to be coupled to a suction source by a first coupler. The ultrasonic tip comprises a shaft and a cutting portion and is removably coupled to the horn by a tip coupler. The shaft comprises a longitudinal axis. The irrigation sleeve has a distal region and a proximal region and defines a lumen. The irrigation sleeve at least partially surrounds the shaft and defines an inlet aperture configured to receive irrigation liquid from an irrigation source. The irrigation sleeve further comprises a first conduit in fluid communication with the lumen. The first conduit is configured to convey irrigation liquid from the inlet aperture to an outlet aperture. The sealing member is positioned between an outer surface of the ultrasonic tip and an inner surface of the lumen. The ultrasonic tip defines a first bore and a second bore. The first bore defines an air inlet disposed proximal to the sealing member when the irrigation sleeve and the ultrasonic tip are coupled to the ultrasonic instrument. The second bore extends from a proximal end of the ultrasonic tip to the first bore. The second bore is in communication with the suction source through the tip coupler. The irrigation sleeve defines a third bore proximal the first bore when the irrigation sleeve and the ultrasonic tip are coupled to the ultrasonic instrument. The ultrasonic surgical assembly defines a path to draw air from an ambient environment through the third bore, then through the first bore, and back through the second bore before the air exits the ultrasonic surgical assembly at the first coupler.

An exemplary method of cutting bone with an ultrasonic tip is disclosed. The method includes providing an ultrasonic tip, which comprises a shaft and a cutting portion and is removably coupled to a horn by a tip coupler. The horn is coupled to a transducer, and the transducer is coupled to a suction source by a first coupler. The shaft comprises a longitudinal axis. The ultrasonic tip defines a first bore defining an air inlet and a second bore extending from a proximal end of the ultrasonic tip to the first bore. The first bore is transverse to the second bore, and the second bore is in communication with the suction source via the tip coupler. The method further includes providing an irrigation sleeve having a distal region and a proximal region and defining a lumen. The irrigation sleeve at least partially surrounds the shaft and is configured to be coupled to an irrigation source with an inlet aperture configured to receive irrigation liquid from the irrigation source. The irrigation sleeve defines a first conduit in fluid communication with the lumen. The first conduit is configured to convey irrigation liquid from the inlet aperture to an outlet aperture. The method further includes drawing air via the suction source through the second bore and the first bore to cool the ultrasonic tip.

An exemplary third ultrasonic surgical assembly is disclosed. The assembly includes an ultrasonic instrument, an ultrasonic tip, and an irrigation sleeve. The ultrasonic instrument includes a housing, a transducer, and a horn. The housing comprises a proximal portion and a distal portion. The transducer is at least partially disposed within the housing. The horn is coupled to the transducer. The ultrasonic tip comprises a shaft and a cutting portion and is removably coupled to the horn. The irrigation sleeve defines a lumen and comprises a body releasably coupled to the distal portion of the housing and has a distal region and a proximal region. The ultrasonic tip further comprises an annular sealing member and defines a groove, with the annular sealing member disposed around the groove, disposed at the proximal region of the body, and positioned between an outer surface of the ultrasonic tip and an inner surface of the lumen when the sleeve and ultrasonic tip are coupled to the ultrasonic instrument. The annular sealing member is configured to prevent movement of fluid proximally to the annular sealing member.

An exemplary fourth ultrasonic surgical assembly is disclosed. The assembly includes an ultrasonic instrument, and an irrigation sleeve. The ultrasonic instrument includes a housing, a transducer, and a horn. The housing comprises a proximal portion and a distal portion. The transducer is at least partially disposed within the housing. The horn is coupled to the transducer. The irrigation sleeve defines a lumen and is configured to be removably coupled to a first ultrasonic tip and a second ultrasonic tip. Each of the first ultrasonic tip and the second ultrasonic tip is configured to be removably coupled to the horn by a tip coupler. Each of the first ultrasonic tip and the second ultrasonic tip comprises a shaft, a cutting portion, and an annular sealing member. The cutting portion of the first ultrasonic tip comprises a cutting geometry that is distinct from a cutting geometry of the cutting portion of the second ultrasonic tip. The first ultrasonic tip defines a first groove positioned at a first distance from the tip coupler of the first ultrasonic tip. The second ultrasonic tip defines a second groove positioned at a second distance from the tip coupler of the second ultrasonic tip. The first distance from the tip coupler of the first ultrasonic tip is not equal to the second distance from the tip coupler of the second ultrasonic tip. Each of the first ultrasonic tip and the second ultrasonic tip includes the annular sealing member disposed around the respective first and second grooves. The irrigation sleeve comprises a body and an irrigation conduit. The body is releasably coupled to the distal portion of the housing. The irrigation conduit is coupled to the body and configured to convey irrigation fluid. The irrigation conduit further defines an inlet aperture disposed at a proximal region of the body, and an outlet aperture disposed at a distal region of the body, wherein irrigation fluid enters the irrigation conduit at the inlet aperture and exits the irrigation conduit at the outlet aperture.

### BRIEF DESCRIPTION OF THE DRAWINGS

Advantages of the present disclosure will be readily appreciated as the same becomes better understood by reference to the following detailed description when considered in connection with the accompanying drawings.
Figure 1 is a top view of an ultrasonic surgical handpiece assembly.
Figure 2 is a sectional view of the ultrasonic surgical handpiece assembly of Figure 1.
Figure 3 is a top view of an ultrasonic tip and an irrigation sleeve with the ultrasonic tip positioned within the irrigation sleeve.
Figure 4A is an exploded view of an ultrasonic tip and irrigation sleeve assembly.
Figure 4B is a sectional view of the ultrasonic tip of Figure 4A at a first cross-sectional area.
Figure 4C is a sectional view of the ultrasonic tip of Figure 4A at a second cross-sectional area.
Figure 5 is sectional view of the irrigation sleeve assembly of Figure 3 in an assembled configuration with the tip inserted therethrough.
Figure 6 is a partial sectional view of the ultrasonic tip, irrigation sleeve, sheath, and irrigation conduit of the irrigation sleeve assembly of Figure 5.
Figure 7 is an alternate sectional view of the irrigation sleeve assembly with the tip inserted therethrough of Figure 3 in an assembled configuration.
Figure 8A is a top view of a distal portion of the ultrasonic tip including a cutting head.
Figure 8B is a side view a distal portion of the ultrasonic tip including a cutting head.

### DETAILED DESCRIPTION

As medical professionals strive for reducing the size of the incisions and the amount of recovery time required following invasive medical procedures, the size of medical instruments used in various medical procedures have become smaller. Many of the medical instruments utilized in performing the various medical procedures may include the use of a cutting accessory, such as an ultrasonic tip. In performing a cutting, shaving, or shaping operation, the cutting accessory will be exposed to varying amounts of force creating stresses within the cutting accessory.

Many of these cutting accessories may also require the use of irrigation or aspiration (*i.e.,* suction) to reduce heat and/or remove debris at the surgical site. One example of a surgical instrument that may utilize irrigation and/or aspiration systems is an ultrasonic surgical handpiece. Generally, one or more lines may be coupled to the ultrasonic surgical handpiece to supply irrigation and/or suction. The ultrasonic surgical handpiece may further comprise a sleeve comprising one or more lumens that may be utilized to direct fluid from an irrigation source toward the surgical site and/or the cutting accessory, *i.e.,* the ultrasonic tip. One such ultrasonic surgical handpiece is described in PCT/US19/52609, entitled "Ultrasonic Surgical Handpiece Assembly".

However, a cutting accessory that is particularly flat or narrow frequently may not include a lumen throughout the entire length of the cutting accessory, *i.e.,* to the cutting edge or blade. Moreover, a higher current within the cutting accessory due to the lack of an aspiration or cooling lumen throughout may cause over-heating of the handpiece. The excessive heat build-up may adversely affects the life span of the handpiece and may also be felt by the surgeon.

Additionally, the shape and geometry of the cutting accessory may affect the amplification factor, or gain, associated with the ultrasonic instrument. By configuring the ultrasonic tip with lower gain in certain implementations, the inventors realized that various benefits may be achieved, including a reduction in stalling, drawing less power to drive the tip, and the ability to drive the tip at a higher current to the ultrasonic handpiece, which may improve cutting rate without an increase in stalling.

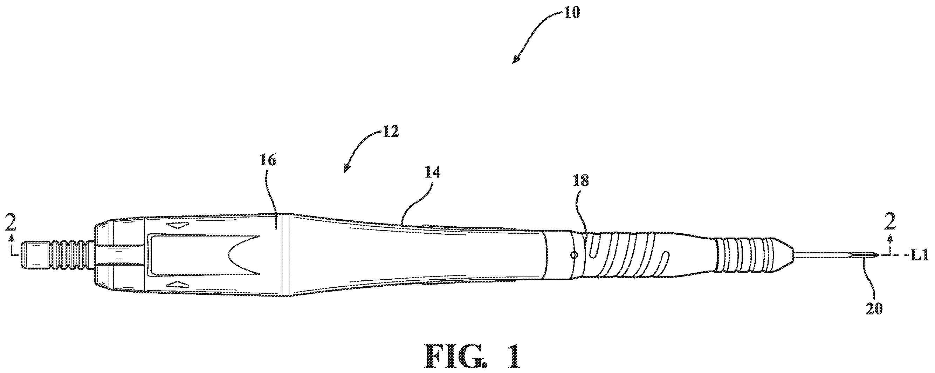

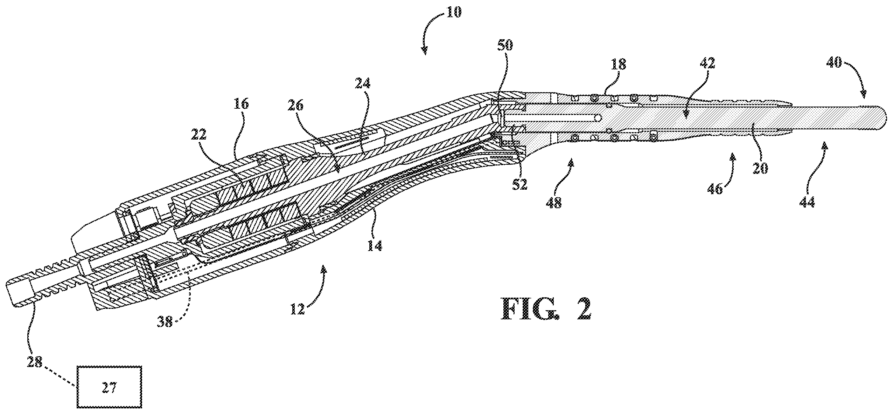

Figures 1 and 2 illustrate an exemplary configuration of an ultrasonic surgical handpiece assembly 10 that may be utilized by a medical professional to remove biological material from a patient. The ultrasonic surgical handpiece assembly 10 may comprise an ultrasonic handpiece 12 including a distal housing portion 14 and a proximal housing portion 16. An irrigation sleeve 18 may be removably coupled to the distal housing portion 14 of the ultrasonic surgical handpiece 12. The irrigation sleeve 18 and an ultrasonic tip 20 may be configured such that the irrigation sleeve 18 surrounds at least a portion of the ultrasonic tip 20 along a length of the ultrasonic tip 20 when both the irrigation sleeve 18 and the ultrasonic tip 20 are coupled to the ultrasonic handpiece 12. The irrigation sleeve 18 and the ultrasonic tip 20 may at least in part comprise an ultrasonic sleeve assembly 14.

Figure 2 illustrates a sectional view of the ultrasonic surgical handpiece assembly 10 of Figure 1. As illustrated in Figure 2, the ultrasonic handpiece 12 may comprise a transducer 22 disposed within a void defined by the distal housing portion 14 and the proximal housing portion 16 of the ultrasonic handpiece 12. The transducer 22 may comprise a plurality of piezoelectric elements or magnetostrictive elements configured to generate mechanical energy.

The ultrasonic handpiece 12 may also comprise a horn 24 that may be at least partially disposed within the void defined by the distal housing portion 14 and the proximal housing portion 16 of the ultrasonic handpiece 12. The horn 24 may comprise a distal end and a proximal end. The proximal end of the horn 24 may be coupled to a distal end of the transducer 22. The transducer 22 may be configured to provide the mechanical energy generated by the piezoelectric element or magnetostrictive element to the horn 24. The horn 24 may also be configured to define a horn lumen 26 that extends from the distal end to the proximal end of the horn 24. The horn lumen 26 may define a portion of a passageway that extends through the ultrasonic handpiece 12 to provide aspiration. The horn lumen 26 has a distal end and a proximal end, and the proximal end may be in communication with a suction source 27). The transducer 22 may be coupled to the suction source 27 via a coupler 28.

The ultrasonic handpiece 12 may further comprise an irrigation line 38 that is disposed within the void defined by the distal housing portion 14 and the proximal housing portion 16 of the ultrasonic handpiece 12. The irrigation line 38 may be configured to extend from the proximal end to the distal end of the ultrasonic handpiece 12. The irrigation line 38 may serve to channel a liquid, such as water or saline from an irrigation system that is coupled to the ultrasonic handpiece 12 through the irrigation sleeve 18. It should be appreciated that the irrigation line 38 may route directly from an irrigation source (not shown) to the irrigation sleeve 18 (*i.e.,* the irrigation line 38 need not be always routed through the handpiece 12).

The ultrasonic tip 20 may comprise a shaft 42 that includes a distal region 44, an intermediate region 46, and a proximal region 48, all disposed along a longitudinal axis L1. The ultrasonic tip 20 may also comprise a coupling feature 50 positioned at the proximal region 48 of the shaft 42 and is configured to couple the proximal region 48 of the ultrasonic tip 20 to the distal end of the horn 24 to allow the horn 24 to be in mechanical communication with the ultrasonic tip 20. The coupling feature 50 may be a threaded coupler 52 configured to engage a corresponding threaded coupler on the distal end of the horn 24. The ultrasonic tip 20 may be threaded into the horn 24 and tightened to a predetermined torque specification to removably secure the ultrasonic tip 20 to the ultrasonic handpiece 12. While not illustrated in the figures, it is contemplated that the coupling feature 50 may be configured as a quick connection, quarter turn fitting, or similar coupling mechanism. It is further contemplated that the coupling feature 50 may be configured to permanently affix the ultrasonic tip 20 to the handpiece 12. For example, the ultrasonic tip 20 may be coupled to the ultrasonic handpiece 12 by a weld, epoxy, or similar coupling method.

Alternatively, it is also contemplated that the ultrasonic tip 20 and the horn 24 may be formed as a unitary component. In such a configuration, the ultrasonic tip 20 may include a base (horn) 24 for coupling to the transducer 22, and a body comprised of the shaft 42 and a cutting portion 40. The body 40, 42 extends from and is coupled to the base 24 at the shaft 42. The body 40, 42 extends from the shaft 42 to the cutting portion 40 along the longitudinal axis L1.

In some aspects, such as the illustrated aspect, the shaft 42 is free of a lumen in the distal region 44. Said differently, the ultrasonic tip may be free of a lumen in the portions of the cutting tip that feature a rectangular shaped cross-sectional area. The shaft 42 may be made of a metal material such as titanium alloy, stainless steel, *etc.* or a non-metallic material such as a composite, depending on the application. In one aspect, the shaft 42 and the cutting portion 40 may be integral, unitary, and one-piece. In another aspect, the cutting portion 40 of the ultrasonic tip 20 may be attached to the shaft 42 by a suitable mechanism such as threads (not shown).

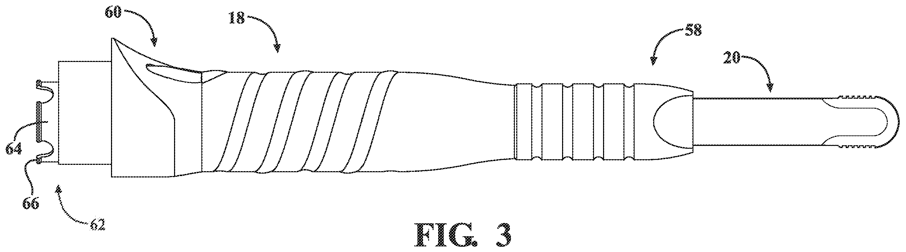

Figure 3 illustrates a top view of an exemplary configuration of the irrigation sleeve 18 having a distal region 58 and a proximal region 60. The irrigation sleeve 18 may comprise an irrigation sleeve coupling mechanism 62 on the proximal region 60 of the irrigation sleeve 18. The irrigation sleeve coupling mechanism 62 may comprise one or more fingers 64 extending proximally from the proximal region 60 of the irrigation sleeve 18. Each of the one or more fingers 64 may comprise a tab 66 extending in a radially outward direction relative to a longitudinal axis L1 of the irrigation sleeve 18. The fingers 64 act as the male fitting configured to couple with a female fitting (not shown) on the ultrasonic handpiece 12 to create a snap-fit or interference fit. It is contemplated that other types of irrigation sleeve coupling mechanisms 62 may be used to couple the irrigation sleeve 18 to the ultrasonic handpiece 12. By way of example and not limitation, the irrigation sleeve coupling mechanisms 62 may be configured as a threaded connection. It should be appreciated that the irrigation sleeve 18 described herein may be used in conjunction with other tip designs than illustrated here.

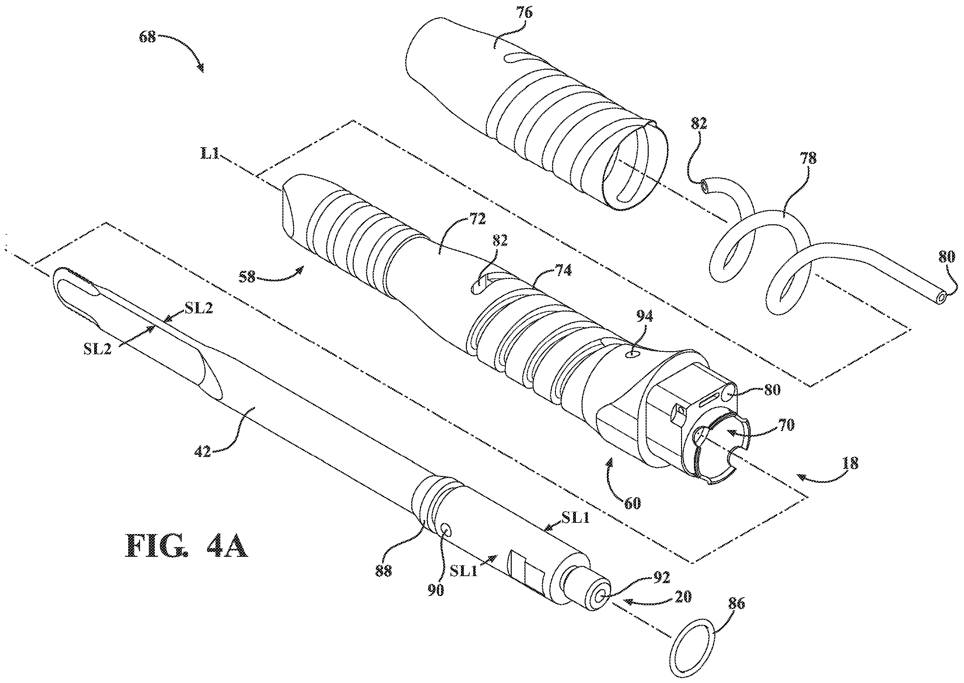

Referring now to Figure 4A, an exploded view an ultrasonic tip and irrigation sleeve assembly 68 is illustrated. The irrigation sleeve assembly 68 includes the irrigation sleeve 18 which defines a lumen 70 extending along the longitudinal axis L1. The irrigation sleeve 18 may have a sleeve body 72 defining a helical groove 74. In an assembled configuration, and when the ultrasonic tip 20 is inserted through the irrigation sleeve 18, the irrigation sleeve 18 may at least partially surround the shaft 42 of the ultrasonic tip 20 when the ultrasonic tip 20 is disposed within the lumen 70 of the irrigation sleeve 18 and the irrigation sleeve 18 is coupled to the handpiece.

The irrigation sleeve assembly 68 may further comprise a sheath 76 having a proximal end and a distal end to correspond with the proximal region 60 and distal region 58 of irrigation sleeve 18, respectively. The sheath 76 may be coupled to and disposed over at least a portion of the sleeve body 72 to surround at least a full revolution of the helical groove 74, but can optionally surround more than two or three revolutions of the helical groove to provide sufficient retention.

The location of helical groove 74 may be different on different tips. By way of example and not limitation, in some configurations, one ultrasonic tip may define a groove positioned at a first distance from a tip coupler (*e.g.*, coupling feature 50) of said tip, whereas a different ultrasonic tip may define a groove positioned at a second distance from a tip coupler (*e.g.*, coupling feature 50) of said tip. In some configurations, the first distance is different from the second distance. This may be useful because different tips may have different node and anti-node locations. It may be useful to position the sealing member align with the node or anti-node of the particular tip.

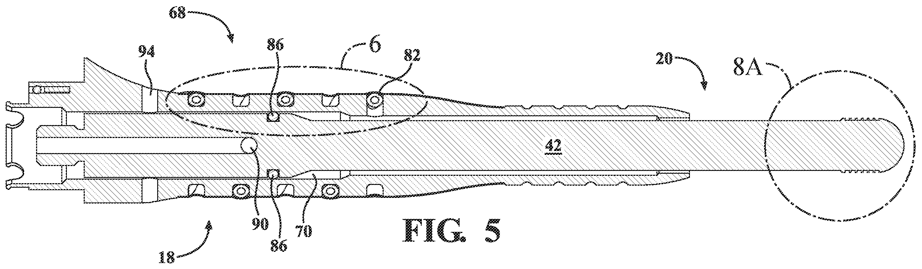

Referring to Figure 5, a sectional view of the irrigation sleeve assembly 68 of Figure 3 in an assembled configuration with the tip inserted therethrough is illustrated. The assembled irrigation sleeve assembly 68 with the ultrasonic tip 20 inserted therethrough shows the ultrasonic tip 20 disposed within the lumen 70 such that the irrigation sleeve 18 partially surrounds the shaft 42.

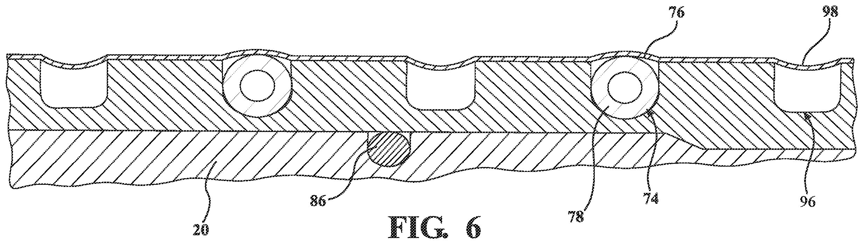

Referring now to Figure 6, a partial sectional view of ultrasonic tip 20, the irrigation sleeve 18, sheath 76, and an irrigation conduit 78 of the irrigation sleeve assembly of Figure 5 is illustrated. The sheath 76 is disposed at least partially over irrigation sleeve 18 to surround at least a full revolution of the helical groove 74, covering the irrigation conduit 78.

In some configurations, the sleeve body 72 may define a second helical groove 96. The second helical groove 96 may be free of the irrigation conduit 78, and may also be partially surrounded by the sheath 76, in other words, one or more revolutions of the second helical groove 96 may be surrounded by the sheath 76.

The sheath 76 may be comprised of a heat-shrink or other suitable material that allows it to deform to the shape of the irrigation conduit 78, as well as the underlying contours of the sleeve body 72, which may include the second helical groove 96. When the sheath 76 is contoured to the second helical groove 96, it may define undulations 98 useful for gripping by a user.

Referring again to Figure 4A, the irrigation sleeve assembly 68 may further comprise the irrigation conduit 78 disposed within the helical groove 74 for conveying irrigation fluid. The irrigation conduit 78 may be in fluid communication with or adjacent to the lumen 70 and may be connected to a liquid source (not shown). The irrigation conduit 78 may define an inlet aperture 80 (disposed at the proximal region 60 of the sleeve body 72 when the irrigation sleeve assembly 68 is in an assembled configuration), and an outlet aperture 82 (disposed at a distal region 58 of the sleeve body 72 when the irrigation sleeve assembly 68 is in an assembled configuration). The irrigation fluid may enter the irrigation conduit 78 at the inlet aperture 80 and exit the irrigation conduit 78 at the outlet aperture 82. In some configurations, the irrigation conduit 78 may comprise a single member defining a continuous length between the inlet aperture 80 and the outlet aperture 82. The irrigation conduit 78 may comprise a flexible material such that the irrigation conduit 78 may be configured to couple directly to a corresponding port of the surgical handpiece 12. In other words, the irrigation conduit 78 may be formed of a material that can be deformed such that the inlet aperture 80 can partially engulf the corresponding port of the ultrasonic handpiece 12 to form the connection to the irrigation source. The helical shape of the groove 74 ensures that the irrigation conduit 78 similarly has a helical shape. This helical shape of the irrigation conduit 78 (tube) and groove 74 provide for a longer fluid return path than a straight return path. This longer path makes it more difficult for fluid to move backward in a proximal direction after it has been initially conveyed through the irrigation conduit 78.

After the fluid has been conveyed through the irrigation conduit 78 distally along the sleeve body 72, the fluid may exit the irrigation conduit 78 at the outlet aperture 82. From there, the fluid may spread to the surface of the sleeve body 72 and/or the shaft 42. In some circumstances, the fluid may return to the aperture 83 and backflow through the irrigation conduit 78. Otherwise, the fluid may return proximally along the sleeve body 72 in a path that flows between an outer diameter of the irrigation conduit 78 and an inner surface of the sleeve body 72 within the sheath 76.

One of the irrigation sleeve 18 and the ultrasonic tip 20 may include a sealing member 86. The sealing member 86 may be an annular sealing member, including, by way of example and not limitation, an O-ring. In some configurations, the sealing member 86 may be coupled to the tip 20. In such an implementation, the sealing member 86 is positioned around a groove 88 defined in an outer surface of the tip 20. This may provide for the sealing member 86 being positioned between the outer surface of the ultrasonic tip 20 and an inner surface of the lumen 70, which may prevent movement of fluid proximal to the sealing member 86 from the outlet aperture 82. Alternatively, the sealing member 86 may be coupled to the sleeve 18, and be positioned to engage the outer surface of the tip 20, again to prevent movement of fluid proximal to the sealing member 86 from the outlet aperture 82.

The sealing member 86 may be also used to dampen the amplitude of vibration of the ultrasonic tip 20. The amplitude of vibration at any point along the ultrasonic tip 20 may depend upon the location along the ultrasonic tip 20 at which vibration is measured. The point along a standing wave where the wave has minimum amplitude is generally referred to as a node. At a node, the vibratory motion is typically minimal. Because the sealing member 86 may be coupled directly to the tip 20, it may be situated anywhere on the ultrasonic tip 20 to decrease transverse motion of the tip 20. In some configurations, it may be suitable to situate the sealing member 86 at or near a node on the tip 20. The location of a node may be different on different ultrasonic tips.

Moreover, because the sealing member 86 may be coupled directly to the tip 20, any sharp surfaces of the ultrasonic tip 20 will never contact the sealing member 86 during placement of the sleeve assembly over the ultrasonic tip 20. Thus, the sealing member 86 is protected from abrasion and other damage when the ultrasonic tip 20 is placed over the sleeve 18. Placement of the sealing member 86 on the ultrasonic tip 20 rather than the irrigation sleeve 18 also prevents the sealing member 86 from spontaneously sliding off or out of the irrigation sleeve assembly 68.

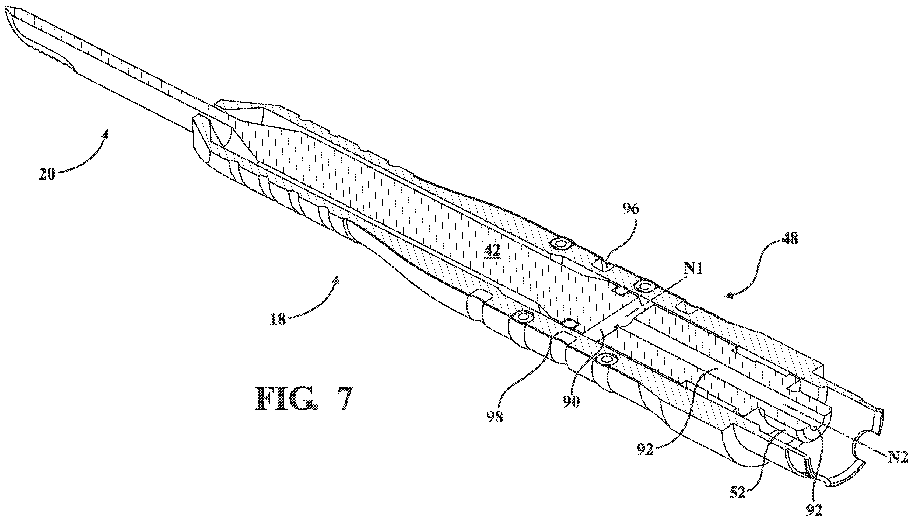

Referring now to Figure 7, an alternate sectional view of the irrigation sleeve assembly with the tip inserted therethrough of Figure 3 in an assembled configuration is illustrated. The ultrasonic tip 20 may define a first bore 90 that behaves as an air inlet. This first bore 90 may be disposed proximal to the sealing member 86 when the irrigation sleeve assembly 68 including the ultrasonic tip 20 are coupled to the handpiece 12. In some configurations, the first bore 90 is located distal the sealing member 86. A second bore 92 may extend from the proximal region 48 of the shaft 42 from the threaded coupler 52 to the first bore 90. In other words, the first bore 90 and the second bore 92 are positioned to be in fluid communication with one another with the first bore 90 transverse the longitudinal axis L1 of the ultrasonic tip 20 and the second bore 92 is concentric with the longitudinal axis L1 of the tip 20. In one example, the axis N1 of the first bore 90 may be perpendicular to an axis N2 of the second bore 92. In some configurations, the first bore 90 may comprise a single bore. In other configurations, such as the configuration illustrated in Figure 7, the first bore 90 may extend laterally from one side of the ultrasonic tip 20 to the other to form a first bore 90 in each side of ultrasonic tip 20. However, in other configurations, more than one first bore 90 may be included in ultrasonic tip 20, wherein each of more than one first bore 90 is separate from one another (but each may still in communication with second bore 92), rather than forming a single channel with a first bore 90 at each end as shown in Figure 7. Furthermore, the first bore 90 need not extend all the way through the tip 20, so long as the first bore 90 extends from the outermost periphery to the second bore 92. If there are a plurality of paths defined from the outer periphery of the ultrasonic tip 20 to the second bore 92, those bores may be arranged in a variety of ways, such as being circumferentially arranged about the surface of the tip 20.

In some configurations, the irrigation sleeve assembly 68 further comprises a third bore 94 proximal the first bore 90 when the irrigation sleeve assembly 68 including the ultrasonic tip 20 are coupled to the handpiece. More particularly, the sleeve body 72 may define third bore 94 to further facilitate air ingress from the ambient environment to the first bore 90. Of course, it is contemplated that the irrigation sleeve assembly 68 may include more than one third bore 94 that defines an aperture to allow ambient air to move from outside the irrigation sleeve assembly 68 to the first bore 90 when the suction source 27 is coupled to the tip 20.

In the illustrated configurations, the inlet aperture 80, the outlet aperture 82, the first bore 90, the second bore 92, and the third bore 94 all have circular cross-sectional shapes. However, in some configurations, all or any combinations of the inlet aperture 80, the outlet aperture 82, the first bore 90, the second bore 92, and/or the third bore 94 may have a shape other than a circular shape, such as various polygonal shapes or elliptical shapes.

The irrigation sleeve 18 may be made from any polymer, for example, a thermoplastic. The distal region 58 of the irrigation sleeve 18 may have a portion of frangible sections that could be cut or snipped to change the length of the irrigation sleeve 18.

Referring again to Figure 5, when the irrigation sleeve assembly 68 is coupled to an irrigation source by a coupler, such as irrigation line 38, it may draw irrigation liquid through the irrigation source via the inlet aperture 80. The irrigation conduit 78 may then convey the irrigation liquid through the inlet aperture 80 to the outlet aperture 82. The conveyance of irrigation liquid through the irrigation sleeve assembly 68 helps provide cooling to the shaft 42 of the ultrasonic tip 20. Additionally, it may help prevent the irrigation sleeve 18 from becoming deformed or melting from excessive heat generated by the motion of the ultrasonic cutting.

In addition, when the irrigation sleeve assembly 68 including the ultrasonic tip 20 are coupled to the handpiece 12, the ultrasonic tip 20 draws air through the third bore 94, then through the first bore 90, back through the second bore 92, and ultimately out through the coupler 28. This movement of air aids in cooling the tip in the area near the sealing member 86, which is prone to overheating due to frictional heat generated by the relative motion between the sealing member 86 and the irrigation sleeve assembly 68.

In addition, the air suction through the tip and/or the sleeve may operates as a smoke evacuation device in some applications. In the illustrated configuration, the ultrasonic tip 20 may be used for lumbar procedures and other relatively "heavy duty" bone-cutting procedures that produce larger amounts of smoke, dust particles, and airborne debris compared to other procedures. The air suction feature may pull some of this from the ambient air surrounding the ultrasonic handpiece 12 and evacuate it through the handpiece 12. The instrument may be used in conjunction with a smoke filter to remove these particulates from the ambient air once aspirated through the tip 20.

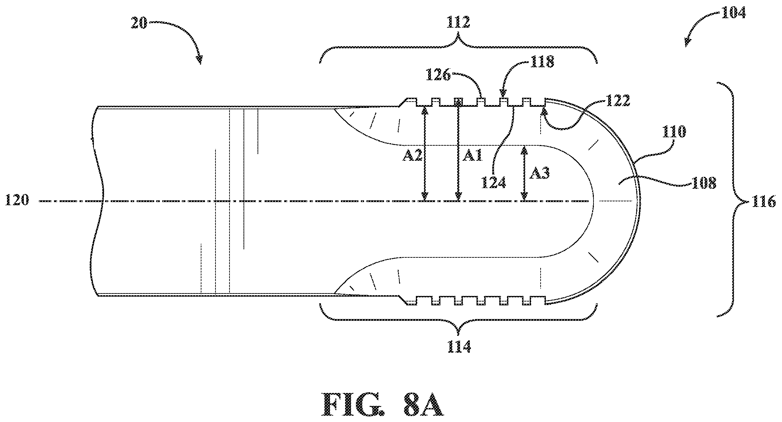

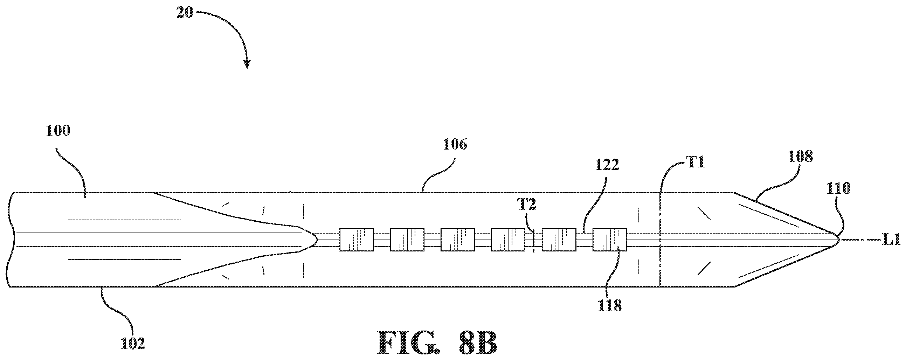

Referring now to Figures 8A-8B, a top view and a side view of a distal portion of the ultrasonic tip including a cutting head are illustrated. The ultrasonic tip 20 may comprise a first side 100 that is substantially planar. The ultrasonic tip 20 may further comprise a second side 102 that is substantially planar that is disposed opposite the first side 100. The ultrasonic tip 20 may further comprise a cutting head (also referred to herein as a "cutting portion") 104 disposed distal the shaft 42. The cutting head 104 may comprise a base portion 106 having a transverse dimension T1 between the first side 100 and the second side 102. The transverse dimension T1 may extend perpendicular to the longitudinal axis L1 of the cutting head 104. The cutting head 104 may further comprise a tapered portion 108 comprising a bevel that extends from the base portion 106 to a cutting edge 110. By way of example and not limitation, in a preferred configuration, the base portion 106 has a transverse dimension of at least 7.975 millimeters. It should be appreciate that other ultrasonic tips may be used with the irrigation sleeve assembly shown above, such as those without the cutting head features described in this application, such as those shown in U.S. Patent Nos. 6,723,110, 6497715, D551764, and 6955680. Similarly, it should be appreciated that the ultrasonic tip may be used with other irrigation sleeve assemblies, such as those shown in PCT/US2019/052609.

The cutting edge 110 comprises a length and may have a U-shaped profile having a first leg portion 112, a second leg portion 114, and an arcuate shaped distal portion 116. By way of example and not limitation, in a preferred configuration, the arcuate shaped distal portion 116 has a thickness of at least 1.35 millimeters. The first leg portion 112 and the second leg portion 114 may be parallel to one another and may each comprise a plurality of cutting teeth, one of which is labeled 118. In some configurations, no more than one half of the length of the cutting edge 110 may have cutting teeth 118. In other configurations, more than one half of the length of the cutting edge 110 may have cutting teeth 118. In some configurations, the arcuate shaped distal portion 116 is free from cutting teeth 118, as is shown in the illustrated configuration in FIGS. 8A-8B.

The cutting portion 40 defines a centerline 120 along the longitudinal axis L1. Moreover, the plurality of cutting teeth 118 comprise at least two adjacent cutting teeth 118 and a notch 122 between each of the adjacent cutting teeth 118. The notch 122 has a transverse dimension T2 along a notch base 124. Each of the cutting teeth 118 has a tooth cutting edge 126. In some configurations, the transverse dimension T2 of the notch 122 is at least 25 times less than the transverse dimension T1 of the base portion 106.

The centerline 120 to the tooth cutting edge 126 defines a first distance A1. The centerline 120 to the notch base 124 defines a second distance A2. The centerline 120 to a starting point of the tapered portion 108 defines a third distance A3. In some configurations, the difference between the first distance A1 and the second distance A2 is less than or equal to 0.25 mm. In some configurations, the first distance A1 is greater than 1.5 times the third distance A3 but is less than 2 times the third distance A3. In the illustrated example, and not by way of limitation, the first distance A1 is 3.4 mm, the second distance A2 is 3.15 mm, and the third distance A3 is 1.85 mm. In this example, the difference between the first distance A1 and the second distance A2 is exactly 0.25 mm. Also, in this example, the first distance A1 is approximately 1.837 times greater than the third distance A3.

The ultrasonic tip 20 may be useful in cutting through both hard and soft tissues. The tooth cutting edges 126 allow for cutting through hard tissue, such as cortical bone. The serrated tooth cutting edges 126 may perform a saw-like operation to cut through denser tissue. By contrast, the cutting edge 110 is a smooth, continuous blade. However, although it may be sharp enough to cut through hard tissue, it may be blunt enough to be atraumatic relative to contacted soft tissue. By way of example and not limitation, the thickness of the cutting edge 110 may be 0.2 mm +/- 0.05 mm. As such, the cutting edge 110 can contact soft tissue with minimal risk of puncturing critical structures, such as the spinal cord. The surgeon will feel a tactile change when the cutting edge 110 moves between hard tissue and soft tissue due to the natural differences in the densities between the different tissue structures, indicating the need to terminate cutting when the cutting edge 110 has reached soft tissue. Thus, the ultrasonic tip 20 offers an advantage over the conventional, sharp bone-cutting blades because it is capable of cutting through hard bone structures with both its teeth edges 126 and the cutting edge 110, while avoiding trauma to underlying soft tissues.

Referring again to Figures 4A-C, different points along the shaft 42 have varying cross-sectional areas. Although transducers and ultrasonic tips are typically designed to have the same resonance frequency in longitudinal resonance devices, a decrease in the cross-sectional area along the length of the ultrasonic tip can amplify the vibrational velocity of the transducer output face. The amplification factor, otherwise known as gain, is determined by the reduction in cross-sectional area, as well as the shape of the ultrasonic tip. Gain can be measured by dividing the tip displacement by the handpiece displacement.

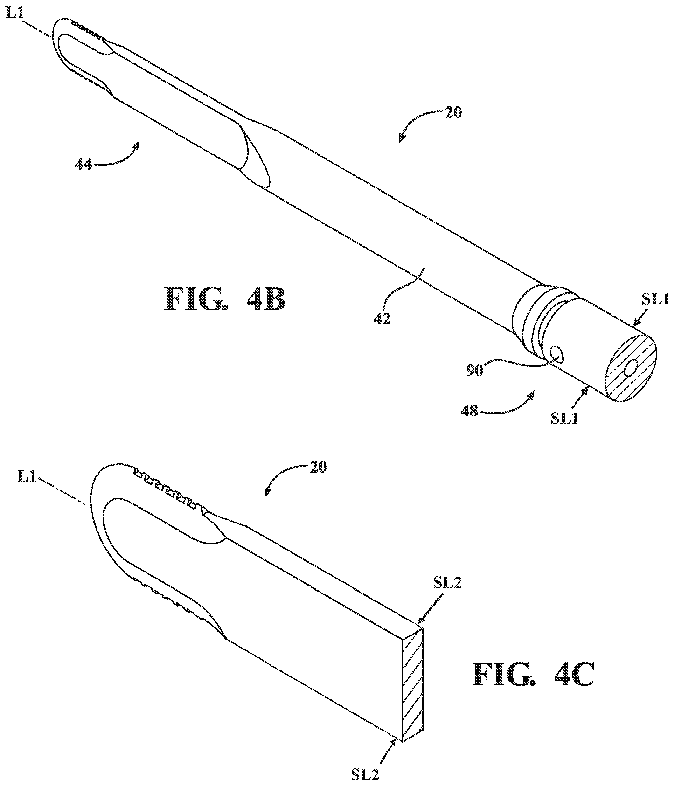

In the illustrated configuration, a cross-sectional area of a first slice SL1 is defined as the largest circular cross-sectional area of the ultrasonic tip 20 and which defines a plane perpendicular to the longitudinal axis L1. Figure 4B illustrates a sectional view of the ultrasonic tip of Figure 4A at the first cross-sectional area. A cross-sectional area of a second slice SL2 is defined at a location is 20 mm proximal to the distal end of the tip. The second slice SL2 defines a plane perpendicular to the longitudinal axis L1, and the second slice SL2 is distal to the first slice SL1. Figure 4C illustrates a sectional view of the ultrasonic tip of Figure 4A at the second slice.

The cross-sectional area of the second slice SL2 is 5 to 6 times smaller than the cross-sectional area of the first slice SL1. In other words, the cross-sectional area of the second slice SL2 is 13-25, 15 to 21, 16-20, or 16.7 - 20 % of the cross-sectional area of the first slice SL1. This results in an ultrasonic tip having a low gain. The low gain of the of the ultrasonic tip 20, which is calculated by dividing the ultrasonic tip 20 displacement by the ultrasonic handpiece 12 displacement, results in a reduction in stalling of the cutting portion 40 (*e.g.,* cutting head 104), which improves performance of the ultrasonic tip 20 overall. It also requires drawing less power to drive the ultrasonic tip 20 and allows for driving the ultrasonic tip 20 at a higher current, which may improve cutting rate without an increase in stalling of the cutting portion 40 (*e.g.,* cutting head 104).

For example and not by way of limitation, in the illustrated configuration, a diameter of the first slice SL1 is 8 mm, with a gun drill hole having a diameter of approximately 2 mm. In this configuration, the cross-sectional area of the first slice SL1 is approximately 47.1 mm². In some configurations, the cross-sectional area of the second slice SL2 may be approximately 8.725 mm². Thus, in this configuration, a value of the cross-sectional area of the second slice SL2 is approximately 5.4 times less than a value of the cross-sectional area of the first slice SL1.

In the illustrated configuration, the gain of the ultrasonic tip 20 is approximately 3. It should be appreciated that the ultrasonic tip 20 may exhibit a gain ranging between 2 and 5, 2 and 4, 2.5 and 3.5, and 2.75 and 3.25. Alternatively, the gain exhibited by the ultrasonic tip 20 may be below 5. By comparison, similar ultrasonic tips known in the art that are used for similar applications have a gain of approximately 7.0-7.4. The low gain of the of the ultrasonic tip 20, which is calculated by dividing the ultrasonic tip 20 displacement by the ultrasonic handpiece 12 displacement, results in a reduction in stalling of the cutting portion 40 (*e.g.,* cutting head 104), which improves performance of the ultrasonic tip 20 overall. It also requires drawing less power to drive the ultrasonic tip 20 and allows for driving the ultrasonic tip 20 at a higher current, which may improve cutting rate without an increase in stalling of the cutting portion 40 (*e.g.,* cutting head 104).

Gain could also be achieved by a uniform outer surface and is composed of two different materials longitudinally aligned with each other such as described in United States Patent No. 9,962,183, entitled "Ultrasonic Torsional Tissue Dissection Utilizing Subaltern Modes of Longitudinal-Torsional Resonators".

The ultrasonic tip 20 may be free of a longitudinal to torsional motion conversion mechanism, and as such, configured to vibrate solely in the longitudinal direction. The ultrasonic tip 20 may consist of titanium.

The irrigation sleeve 18 described herein may be used with any type of ultrasonic tip. In other words, the irrigation sleeve 18 described herein may be used with ultrasonic tips that do not include the first and second bores 90, 92 described. In addition, the irrigation sleeve 18 described herein may be used with ultrasonic tips that do not having a cutting head that have two substantially planar sides. For example, the irrigation sleeve 18 described herein may be used with ultrasonic tips that have cylindrical shapes and that rely on longitudinal, torsion, or longitudinal and torsional motion, such as those described in United States Patent Publication No. 2005/0177184, United States Patent No. 8,512,340, United States Patent Publication No. 2008/0208231.

The instrument described herein may be used with any of the tip configurations and/or any of the irrigation sleeve configurations described herein.

Several configurations have been discussed in the foregoing description. However, the configurations discussed herein are not intended to be exhaustive or limit the invention to any particular form. For example, while the example configurations describe the surgical instrument as an ultrasonic handpiece, it is further contemplated that the features and concepts described with regard to the ultrasonic handpiece may be applied to other medical or surgical instruments. This similarly applies to the ultrasonic tip, which may further include blades, drill bits, rotating burs, open-window shavers, and the like. The terminology which has been used is intended to be in the nature of words of description rather than of limitation.

## Claims

1. An irrigation sleeve (18) for an ultrasonic surgical assembly (10), the ultrasonic surgical assembly (10) comprising a housing (12) having a proximal portion (16) and a distal portion (14), a transducer (22) at least partially disposed within the housing (12), a horn (24) coupled to the transducer (22), and an ultrasonic tip (20) comprising a shaft (42), the ultrasonic tip (20) removably coupled to the horn (24), the irrigation sleeve (18) comprising:
a body (72) having a distal region and a proximal region;
a sheath (76) coupled to and disposed over a portion of said body (72), said sheath (76) having a proximal end and an opposing distal end; and
an irrigation conduit (78) for conveying irrigation fluid,
the irrigation conduit (78) defining an inlet aperture (80) disposed at the proximal region of said body (72), and an outlet aperture (82), wherein the irrigation fluid is configured to enter the irrigation conduit (78) at the inlet aperture (80) and exit the irrigation conduit (78) at the outlet aperture (82);
**characterized in that**
the body (72) is releasably coupleable to the distal portion (14) of the housing (12), said body (72) defining a helical groove (74) which at least partially surrounds the shaft (42) of the ultrasonic tip (20) when the body (72) is coupled to the distal portion (14) of the housing (12) such that the ultrasonic tip (20) is in a lumen (70) defined by the irrigation sleeve (18);
the sheath (76) is coupled to and disposed over a portion of said body (72) to surround at least a full revolution of the helical groove (74); and
the irrigation conduit (78) is disposed within said helical groove (74), wherein the outlet aperture (82) is disposed at a distal region of said body (72).

2. The irrigation sleeve (18) of claim 1, wherein said body (72) defines a second helical groove (96) free of the irrigation conduit (78) and surrounded by said sheath (76), and wherein the second helical groove (96) covered by the sheath (76) defines a gripping surface for a user.

3. The irrigation sleeve (18) of any one of claims 1 and 2, wherein said irrigation conduit (78) comprises of a flexible material, said irrigation conduit (78) comprising a single member defining a continuous length between said inlet aperture (80) and said outlet aperture (82).

4. An ultrasonic surgical assembly (10) comprising:
an ultrasonic instrument comprising:
a housing (12) comprising a proximal portion (16) and a distal portion (14),
a transducer (22) at least partially disposed within said housing (12), and
a horn (24) coupled to said transducer (22);
an ultrasonic tip (20) comprising a shaft (42), the ultrasonic tip (20) removably coupled to said horn (24); and
the irrigation sleeve (18) of any one of claims 1-3.

5. The ultrasonic surgical assembly (10) of claim 4, further comprising an annular sealing member (86) disposed at the proximal region of said body (72) and coupled to an outer surface of said ultrasonic tip (20), wherein the annual sealing member (86) is positioned between the outer surface of said ultrasonic tip (20) and an inner surface of said lumen (70) and configured to prevent movement of fluid proximally to said annular sealing member (86).

6. The ultrasonic surgical assembly (10) of claim 5, wherein the ultrasonic tip (20) defines:
a first bore (90) defining an air inlet disposed proximal to the annular sealing member (86), and
a second bore (92) extending from the proximal end of the shaft (42) to the first bore (90) to form a fluid path between the first bore (90) and the second bore (92).

7. The ultrasonic surgical assembly (10) of claim 6, wherein the ultrasonic tip (20) further comprises:
a cutting portion (40);
a first side (100) that is substantially planar extending from a proximal end to a distal end; and
a second side (102) that is substantially planar and disposed opposite the first side (100), and extending from the proximal end to the distal end,
the cutting portion (40) including a cutting head (104) disposed at the distal end.

8. The ultrasonic surgical assembly (10) of any one of claims 6 and 7, wherein said irrigation sleeve (18) further comprises a third bore (94) proximal the first bore (90) when the irrigation sleeve (18) and the ultrasonic tip (20) are coupled to the ultrasonic instrument.

9. The ultrasonic surgical assembly (10) of any one of any one of claims 5-8, wherein said ultrasonic tip (20) further defines a groove (88), and wherein said sealing member (86) is disposed around said groove (88).

10. The ultrasonic surgical assembly (10) of any one of claims 6-8, wherein said first bore (90) is perpendicular to an axis (N2) of said second bore (92).

11. The ultrasonic surgical assembly (10) of any one of claims 7 to 10, as far as dependent on claim 7, wherein said ultrasonic tip (20) further comprises:
a base (24) for coupling to a transducer (22); and
a body (40, 42) extending from the base (24), the body (40, 42) comprising the shaft (42) and the cutting portion (40), wherein the body (40, 42) is coupled to the base (24) at the shaft (42) and said body (40, 42) extends from said shaft (42) to said cutting portion (40) along the longitudinal axis (L1),
wherein the cutting head (104) comprises a base portion (106) and a tapered portion (108).

12. The ultrasonic surgical assembly (10) of claim 11, wherein:
said base portion (106) has a transverse dimension between the first side (100) and the second side (102), the transverse dimension extending perpendicular to the longitudinal axis (L1),
said tapered portion (108) comprises a bevel and extends from the base portion (106) to a cutting edge (110), said cutting edge (110) comprising a length and having a U-shaped profile having a first leg portion (112), a second leg portion (114), and an arcuate shaped distal portion (116), and
the first leg portion (112) and the second leg portion (114) are parallel to one another and each comprises a plurality of cutting teeth (118).

13. The ultrasonic surgical assembly (10) of any one of claims 7, 8, 11, and 12, wherein said shaft (42) of said ultrasonic tip (20) is free of a lumen in the distal end.

## Patentansprüche

1. Bewässerungshülse (18) für eine chirurgische Ultraschallanordnung (10), wobei die chirurgische Ultraschallanordnung (10) ein Gehäuse (12) mit einem proximalen Abschnitt (16) und einem distalen Abschnitt (14), einen Wandler (22), der zumindest teilweise innerhalb des Gehäuses (12) angeordnet ist, ein mit dem Wandler (22) gekoppeltes Horn (24) und eine Ultraschallspitze (20) mit einem Schaft (42) umfasst, wobei die Ultraschallspitze (20) abnehmbar mit dem Horn (24) gekoppelt ist, und wobei die Bewässerungshülse (18) umfasst:
einen Körper (72) mit einem distalen Bereich und einem proximalen Bereich;
eine Hülle (76), die mit einem Abschnitt des Körpers (72) gekoppelt und darüber angeordnet ist, wobei die Hülle (76) ein proximales Ende und ein gegenüberliegendes distales Ende aufweist; und
eine Bewässerungsleitung (78) zum Fördern einer Bewässerungsflüssigkeit, wobei die Bewässerungsleitung (78) eine Einlassöffnung (80), die am proximalen Bereich des Körpers (72) angeordnet ist, und eine Auslassöffnung (82) definiert, wobei die Bewässerungsflüssigkeit dazu ausgebildet ist, an der Einlassöffnung (80) in die Bewässerungsleitung (78) einzutreten und an der Auslassöffnung (82) aus der Bewässerungsleitung (78) auszutreten;
**dadurch gekennzeichnet, dass**
der Körper (72) lösbar mit dem distalen Abschnitt (14) des Gehäuses (12) koppelbar ist, wobei der Körper (72) eine spiralförmige Nut (74) definiert, die zumindest teilweise den Schaft (42) der Ultraschallspitze (20) umgibt, wenn der Körper (72) mit dem distalen Abschnitt (14) des Gehäuses (12) gekoppelt ist, so dass sich die Ultraschallspitze (20) in einem Lumen (70) befindet, das durch die Bewässerungshülse (18) definiert ist;
die Hülle (76) mit einem Abschnitt des Gehäuses (12) gekoppelt und über diesem angeordnet ist, um zumindest eine volle Umdrehung der spiralförmigen Nut (74) zu umgeben; und
die Bewässerungsleitung (78) innerhalb der spiralförmigen Nut (74) angeordnet ist, wobei die Auslassöffnung (82) in einem distalen Bereich des Körpers (72) angeordnet ist.

2. Bewässerungshülse (18) nach Anspruch 1, wobei der Körper (72) eine zweite spiralförmige Nut (96) definiert, die frei von der Bewässerungsleitung (78) und von der Hülle (76) umgeben ist, und wobei die zweite spiralförmige Nut (96), die von der Hülle (76) bedeckt ist, eine Griffläche für einen Benutzer definiert.

3. Bewässerungshülse (18) nach einem der Ansprüche 1 und 2, wobei die Bewässerungsleitung (78) ein flexibles Material umfasst und die Bewässerungsleitung (78) ein einzelnes Element umfasst, das eine durchgehende Länge zwischen der Einlassöffnung (80) und der Auslassöffnung (82) definiert.

4. Chirurgische Ultraschallanordnung (10), umfassend:
ein Ultraschallinstrument, umfassend:
ein Gehäuse (12), das einen proximalen Abschnitt (16) und einen distalen Abschnitt (14) umfasst,
einen Wandler (22), der zumindest teilweise innerhalb des Gehäuses (12) angeordnet ist, und
ein Horn (24), das mit dem Wandler (22) gekoppelt ist;
eine Ultraschallspitze (20), die einen Schaft (42) umfasst, wobei die Ultraschallspitze (20) abnehmbar mit dem Horn (24) gekoppelt ist; und
die Bewässerungshülse (18) nach einem der Ansprüche 1-3.

5. Chirurgische Ultraschallanordnung (10) nach Anspruch 4, ferner umfassend ein ringförmiges Dichtungselement (86), das im proximalen Bereich des Körpers (72) angeordnet und mit einer Außenfläche der Ultraschallspitze (20) gekoppelt ist, wobei das ringförmige Dichtungselement (86) zwischen der Außenfläche der Ultraschallspitze (20) und einer Innenfläche des Lumens (70) positioniert und dazu ausgebildet ist, eine Bewegung von Flüssigkeit proximal zu dem ringförmigen Dichtungselement (86) zu verhindern.

6. Chirurgische Ultraschallanordnung (10) nach Anspruch 5, wobei die Ultraschallspitze (20) definiert:
eine erste Bohrung (90), die einen Lufteinlass definiert, der proximal zu dem ringförmigen Dichtungselement (86) angeordnet ist, und
eine zweite Bohrung (92), die sich von dem proximalen Ende des Schafts (42) zu der ersten Bohrung (90) erstreckt, um einen Flüssigkeitspfad zwischen der ersten Bohrung (90) und der zweiten Bohrung (92) auszubilden.

7. Chirurgische Ultraschallanordnung(10) nach Anspruch 6, wobei die Ultraschallspitze (20) ferner umfasst:
einen Schneidabschnitt (40);
eine erste Seite (100), die im Wesentlichen planar ist und sich von einem proximalen Ende zu einem distalen Ende erstreckt; und
eine zweite Seite (102), die im Wesentlichen planar und gegenüber der ersten Seite (100) angeordnet ist und sich von dem proximalen Ende zu dem distalen Ende erstreckt,
wobei der Schneidabschnitt (40) einen Schneidkopf (104) aufweist, der an dem distalen Ende angeordnet ist.

8. Chirurgische Ultraschallanordnung (10) nach einem der Ansprüche 6 und 7, wobei die Bewässerungshülse (18) ferner eine dritte Bohrung (94) proximal zu der ersten Bohrung (90) umfasst, wenn die Bewässerungshülse (18) und die Ultraschallspitze (20) mit dem Ultraschallinstrument gekoppelt sind.

9. Chirurgische Ultraschallanordnung (10) nach einem der Ansprüche 5 bis 8, wobei die Ultraschallspitze (20) ferner eine Nut (88) definiert und wobei das Dichtungselement (86) um die Nut (88) herum angeordnet ist.

10. Chirurgische Ultraschallanordnung (10) nach einem der Ansprüche 6 bis 8, wobei die erste Bohrung (90) senkrecht zu einer Achse (N2) der zweiten Bohrung (92) verläuft.

11. Chirurgische Ultraschallanordnung (10) nach einem der Ansprüche 7 bis 10, sofern abhängig von Anspruch 7, wobei die Ultraschallspitze (20) ferner umfasst:
eine Basis (24) zur Kopplung mit einem Wandler (22); und
einen Körper (40, 42), der sich von der Basis (24) aus erstreckt, wobei der Körper (40, 42) den Schaft (42) und den Schneidabschnitt (40) umfasst, wobei der Körper (40, 42) an dem Schaft (42) mit der Basis (24) gekoppelt ist und der Körper (40, 42) sich von dem Schaft (42) zu dem Schneidabschnitt (40) entlang der Längsachse (L1) erstreckt,
wobei der Schneidkopf (104) einen Basisabschnitt (106) und einen sich verjüngenden Abschnitt (108) umfasst.

12. Chirurgische Ultraschallanordnung (10) nach Anspruch 11, wobei:
der Basisabschnitt (106) eine Querabmessung zwischen der ersten Seite (100) und der zweiten Seite (102) aufweist, die sich senkrecht zu der Längsachse (L1) erstreckt,
der sich verjüngende Abschnitt (108) eine Abschrägung umfasst und sich von dem Basisabschnitt (106) zu einer Schneidkante (110) erstreckt, wobei die Schneidkante (110) eine Länge umfasst und ein U-förmiges Profil mit einem ersten Schenkelabschnitt (112), einem zweiten Schenkelabschnitt (114) und einem bogenförmigen distalen Abschnitt (116) aufweist, und
der erste Schenkelabschnitt (112) und der zweite Schenkelabschnitt (114) parallel zueinander sind und jeweils eine Mehrzahl von Schneidzähnen (118) umfassen.

13. Chirurgische Ultraschallanordnung (10) nach einem der Ansprüche 7, 8, 11 und 12, wobei der Schaft (42) der Ultraschallspitze (20) in dem distalen Ende kein Lumen aufweist.

## Revendications

1. Manchon d'irrigation (18) pour un ensemble chirurgical à ultrasons (10), l'ensemble chirurgical à ultrasons (10) comprenant un boîtier (12) doté d'une partie proximale (16) et d'une partie distale (14), un transducteur (22) disposé, au moins en partie, dans le boîtier (12), une partie conique (24) couplée au transducteur (22), et un embout ultrasonique (20) comprenant une tige (42), l'embout ultrasonique (20) étant couplé de manière amovible à la partie conique (24), le manchon d'irrigation (18) comprenant:
un corps (72) ayant une région distale et une région proximale;
une gaine (76) couplée à une partie dudit corps (72) et disposée sur cette dernière, ladite gaine (76) ayant une extrémité proximale et une extrémité distale opposée; et
un conduit d'irrigation (78) pour l'acheminement du liquide d'irrigation,
le conduit d'irrigation (78) définissant une ouverture d'entrée (80) disposée dans la région proximale dudit corps (72), et une ouverture de sortie (82), dans lequel le liquide d'irrigation étant adapté pour entrer dans le conduit d'irrigation (78) au niveau de l'ouverture d'entrée (80) et sortir du conduit d'irrigation (78) au niveau de l'ouverture de sortie (82);
**caractérisé en ce que**
le corps (72) peut être accouplé de manière amovible à la partie distale (14) du boîtier (12), ledit corps (72) définissant une rainure hélicoïdale (74) qui entoure, au moins à certains endroits, la tige (42) de l'embout ultrasonique (20) lorsque le corps (72) est accouplé à la partie distale (14) du boîtier (12) de telle sorte que l'embout ultrasonique (20) se trouve dans une lumière (70) définie par le manchon d'irrigation (18);
la gaine (76) est accouplée à une partie dudit corps (72) et disposée sur ce dernier permettant d'entourer la rainure hélicoïdale (74) en faisant au moins un tour complet; et
le conduit d'irrigation (78) est disposé à l'intérieur de ladite rainure hélicoïdale (74), l'ouverture de sortie (82) étant ménagée dans une région distale dudit corps (72).

2. Manchon d'irrigation (18) selon la revendication 1, dans lequel ledit corps (72) définit une seconde rainure hélicoïdale (96) sans conduit d'irrigation (78) et entourée par ladite gaine (76), et dans lequel la seconde rainure hélicoïdale (96) recouverte par la gaine (76) définit une surface de préhension destinée à l'utilisateur.

3. Manchon d'irrigation (18) selon l'une quelconque des revendications 1 et 2, dans lequel ledit conduit d'irrigation (78) est constitué d'un matériau flexible, ledit conduit d'irrigation (78) comprenant un seul élément définissant une longueur continue entre ladite ouverture d'entrée (80) et ladite ouverture de sortie (82).

4. Ensemble chirurgical à ultrasons (10) comprenant:
un instrument à ultrasons comprenant:
un logement (12) comprenant une partie proximale (16) et une partie distale (14);
un transducteur (22) disposé, au moins en partie, à l'intérieur dudit boîtier (12), et
une partie conique (24) accouplée au corps (22);
un embout ultrasonique (20) comprenant une tige (42), l'embout ultrasonique (20) étant couplé de manière amovible audit élément conique (24); et
le manchon d'irrigation (18) selon l'une quelconque des revendications 1 à 3.

5. Ensemble chirurgical à ultrasons (10) selon la revendication 4, comprenant en outre un élément d'étanchéité annulaire (86) disposé dans la région proximale dudit corps (72) et accouplé à une surface extérieure dudit embout ultrasonique (20), l'élément d'étanchéité annulaire (86) étant positionné entre la surface extérieure dudit embout ultrasonique (20) et une surface intérieure de ladite lumière (70) et conçu pour empêcher que le liquide ne se déplace à proximité dudit élément d'étanchéité annulaire (86).

6. Ensemble chirurgical à ultrasons (10) selon la revendication 5, dans lequel l'embout ultrasonique (20) définit:
un premier trou (90) définissant une entrée d'air disposée à proximité de l'élément d'étanchéité annulaire (86), et
un deuxième trou (92) s'étendant de l'extrémité proximale de la tige (42) au premier trou (90) pour former un passage de liquide entre le premier trou (90) et le deuxième trou (92).

7. Ensemble chirurgical à ultrasons (10) selon la revendication 6, dans lequel l'embout ultrasonique (20) comprend en outre:
une partie de coupe (40);
un premier côté (100) sensiblement plan qui s'étend d'une extrémité proximale à une extrémité distale; et
un second côté (102) qui est sensiblement plan et disposé en regard du premier côté (100), et qui s'étend de l'extrémité proximale à l'extrémité distale,
la partie de coupe (40) comprend une tête de coupe (104) disposée à l'extrémité distale.

8. Ensemble chirurgical à ultrasons (10) selon l'une quelconque des revendications 6 et 7, dans lequel ledit manchon d'irrigation (18) comprend en outre un troisième trou (94) à proximité du premier trou (90) lorsque le manchon d'irrigation (18) et l'embout ultrasonique (20) sont accouplés à l'instrument à ultrasons.

9. Ensemble chirurgical à ultrasons (10) selon l'une quelconque des revendications 5 à 8, dans lequel ledit embout ultrasonique (20) délimite en outre une rainure (88), et dans lequel ledit élément d'étanchéité (86) est disposé autour de ladite rainure (88).

10. Ensemble chirurgical à ultrasons (10) selon l'une quelconque des revendications 6 à 8, dans lequel le premier trou (90) est perpendiculaire à l'axe (N2) du deuxième trou (92).

11. Ensemble chirurgical à ultrasons (10) selon l'une quelconque des revendications 7 à 10, dans la mesure où elle dépend de la revendication 7, dans lequel ledit embout ultrasonique (20) comprend en outre:
une base (24) pour accoupler un transducteur (22); et
un corps (40, 42) s'étendant à partir de la base (24), le corps (40, 42) comprenant la tige (42) et la partie de coupe (40), dans lequel le corps (40, 42) est accouplé à la base (24) au niveau de la tige (42) et ledit corps (40, 42) s'étend depuis ladite tige (42) à la partie de coupe (40) le long de l'axe longitudinal (L1),
dans lequel la tête de coupe (104) comprend une partie de base (106) et une partie conique (108).

12. Ensemble chirurgical à ultrasons (10) selon la revendication 11, dans lequel:
ladite partie de base (106) présente une dimension transversale entre le premier côté (100) et le second côté (102), la dimension transversale s'étendant perpendiculairement à l'axe longitudinal (L1),
ladite partie conique (108) comprend un biseau et s'étend de la partie de base (106) à un bord de coupe (110), ledit bord de coupe (110) comprenant une longueur et ayant un profil en forme de U avec une première partie pied (112), une deuxième partie pied (114), et une partie distale en forme d'arc (116), et
la première partie pied (112) et la deuxième partie pied114) sont parallèles l'une à l'autre et comprennent chacune une pluralité de dents de coupe (118).

13. Ensemble chirurgical à ultrasons (10) selon l'une quelconque des revendications 7, 8, 11, et 12, dans lequel ladite tige (42) dudit embout ultrasonique (20) est exempte de lumière à l'extrémité distale.
